# EUROPEAN PATENT SPECIFICATION

(11) **EP 4 445 941 B1**
(45) Date of publication and mention of the grant of the patent: **20.05.2026**
(21) Application number: 24166754.2
(22) Date of filing: 27.03.2024
(51) Int. Cl.: A61M 39/26, A61M 39/10

(54) **VALVE CONNECTOR FOR MEDICAL LINES**
VENTILVERBINDER FÜR MEDIZINISCHE LEITUNGEN
RACCORD DE SOUPAPE POUR CONDUITES MÉDICALES

(30) Priority: 12.04.2023 IT 202300006996
(43) Date of publication of application: 16.10.2024
(73) Proprietor: Industrie Borla S.p.A., 10024 Moncalieri (Torino) (IT)
(72) Inventor: GUALA, Gianni, I-10133 Torino (IT)
(74) Representative: Buzzi, Notaro & Antonielli d'Oulx S.p.A.

(56) References cited:
- US-A1- 2003 209 681
- US-A1- 2007 218 757
- US-A1- 2008 190 485
- US-A1- 2011 046 573

## Description

### Field of the invention

The present invention relates to valve connectors for medical lines, for example infusion lines through an introducer for a fluid infusion substance, typically a Luer or Luer-Lock connector for example of a needleless syringe.

### State of the prior art

Document EP-1834665A1 in the name of the Applicant discloses a valve connector according to the preamble of claim 1, comprising an axially elongated tubular body formed by a base and a lid coaxially joined together and defining an inlet and an outlet. The base is formed coaxially with an integral hollow pin communicating with the outlet and having a closed terminal protruding into the lid and having a lateral passage adapted to be placed in communication with the inlet. An elastic element is inserted coaxially along the lid and it includes an elastic head having a radial pre-slit and normally arranged in a closing condition in the inlet, in which said pre-slit is closed. The elastic head can be displaced axially towards the closed terminal of the hollow pin, following the insertion of a fluid introducer into the inlet, to allow the pre-slit to take an opening condition. The median part of the elastic element consists of a tubular stem joined to the head and having an internal annular gasket in sliding contact with the hollow pin to control the communication between the lateral passage of the closed terminal of the hollow pin and the inlet. Furthermore, the elastic element is formed with an end retaining part having an end edge blocked between the base and the lid and joined to the tubular stem through a section which is elastically deformable under traction during the axial displacement of the elastic head from the closing condition towards the opening condition.

This connector according to European patent application EP-1834665A1 is easily cleanable and disinfectable ("swabbable") on the inlet end side, and it satisfactorily meets several crucial requirements as pertains to use thereof in the medical industry.

Firstly, it is appropriate to guarantee an effective sealed closure of the inlet end of the tubular body carried out by the head of the elastic element, therefore ensuring a full anti-bacterial barrier even following repeated openings and closures of the valve connector.

Secondly, the opening and closure operation of the communication between the inlet and the outlet of the connector upon the insertion and respectively the removal of the introducer is reliable and repeatable, without risks of malfunctions which could lead to severe risks for the patient connected to the valve connector. This also stems from the fact that the number of moving mechanical members is reduced to the least possible.

Thirdly, this known connector is capable of satisfactorily bearing any non-excessive overpressure which can be generated therein in use, and in the closing or deactivated condition it guarantees an effective sealing to positive and negative pressure.

In order to increase the ability of the connector to operate reliably with higher internal overpressure, even in the order of 100 psi and above, in document EP-2123322A1 the Applicant envisaged providing the connector with a substantially inextensible annular member which surrounds an area of the tubular stem of the elastic hollow element and is axially slidable in the lid of the tubular body during the displacement of the elastic head between the aforementioned closing and opening conditions.

Despite being entirely satisfactory from the connector operation point of view, this solution entails construction challenges in relation to the moulding of the components thereof, in particular of the base with the integrally joined hollow pin, as well as the assembling thereof.

### Summary of the invention

The present invention represents an improvement of the valve connector known from the aforementioned documents EP-1834665A1 and EP-2123322A1, and in particular it has the purpose of simplifying the structure and therefore the manufacturing of the valve connector while keeping intact, and even improving it, the ability thereof to ensure the hermetic sealing even in the event of internal overpressure widely exceeding values in the order of 100 psi, without using additional components.

A further object of the invention is to provide a valve connector having particularly small dimensions and provided with improved flexibility of use.

These objects are essentially attained due to the combination of the following peculiar characteristics:
- the elastic head, in the condition for opening said pre-slit, remains axially spaced from said closed terminal of the hollow pin of the base,
- the lateral passage of said closed terminal consists of a plurality of radial slots separated from each other by external slides cooperating with said internal annular gasket of the tubular stem of the elastic element to open the communication between the inlet and the outlet of the connector when said elastic head moves to said opening condition,
- the end retaining part of the elastic element is formed with a convolute which - during the displacement of said elastic head from said closing condition towards said opening condition - deforms under traction within an annular space of the base.

Thanks to this configuration, the connector according to the invention is optimised not only from a construction point of view, given that the manufacturing thereof is significantly simplified, but also from an operation point of view both in relation to the activation and deactivation promptness and reliability both with regard to the ability to ensure the hermetic sealing thereof up to internal overpressure even exceeding values in the order of 100 psi and above.

The distance of the elastic head from the closed terminal of the hollow pin of the base allows the valve connector according to the invention to be activated by luer-cone introducers even longer than the standard length and even with luer slip-type syringes, which expands the flexibility of use of the connector.

The particular conformation of the end retaining part of the elastic element ensures, during the step for activating the valve connector, the most correct elastic deformation thereof without "buckling" effects.

The specific configuration of the base of the connector, and in particular the arrangement of the lateral slots of the integrally joined hollow pin, simplifies the injection moulding thereof avoiding the use of complex lateral carriages.

### Brief description of the drawings

The invention will now be described in detail, purely by way of non-limiting example, with reference to the attached drawings, wherein:

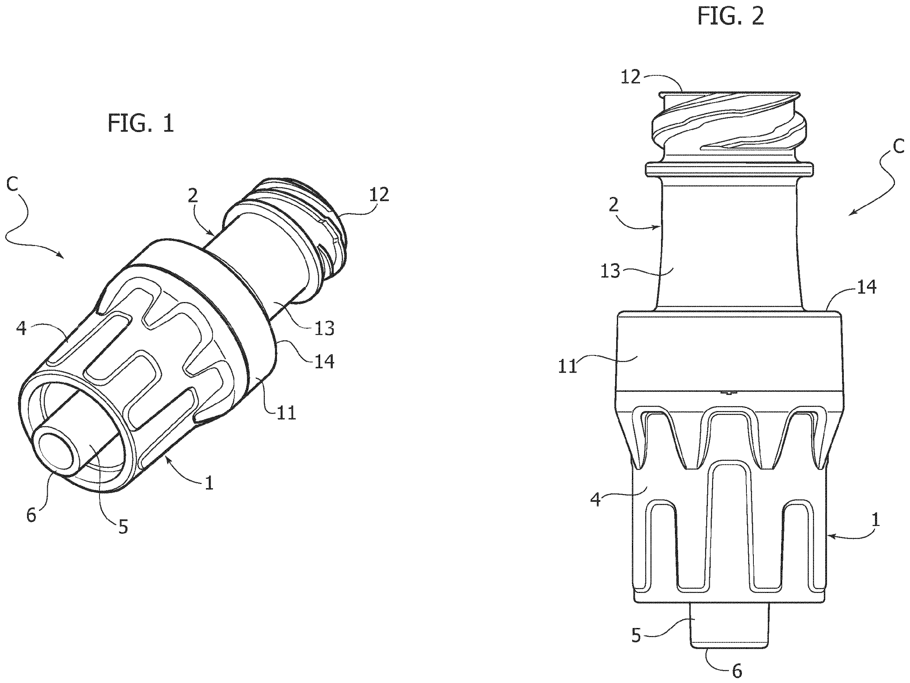

- figure 1 is a schematic perspective view of the valve connector according to the invention,
- figure 2 is a side elevational view and a view in larger scale of the valve connector,
- figure 3 is an axial cross-sectional view of figure 2 which represents the valve connector in deactivated condition that is closing condition,
- figure 4 is a top plan view of figure 2,

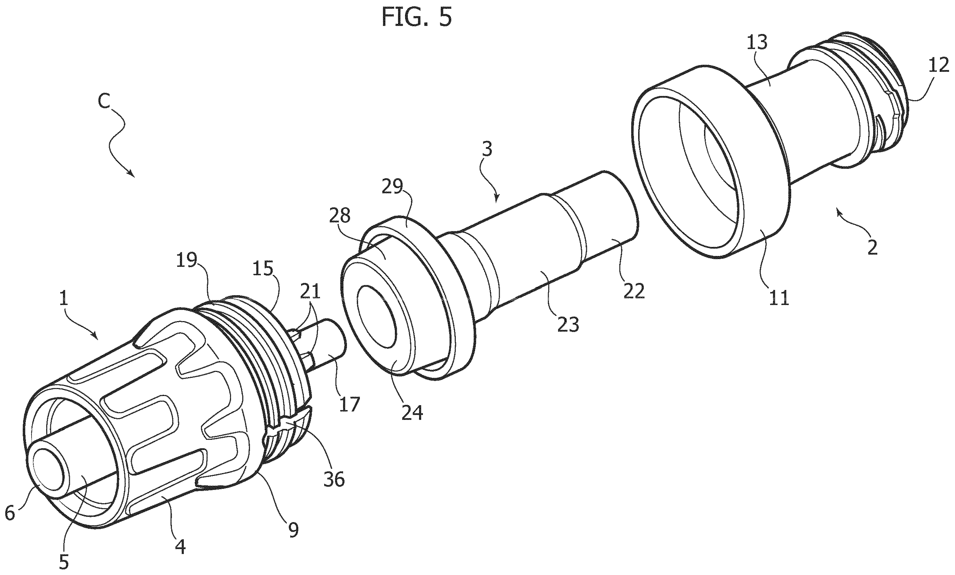

- figure 5 is an exploded view of figure 1,

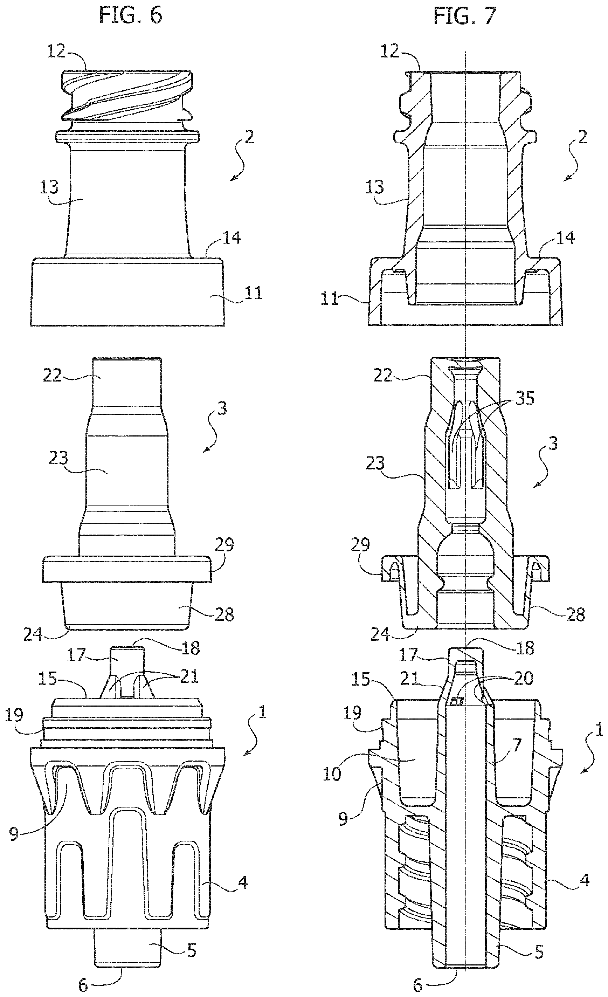

- figure 6 is an exploded view of figure 2,
- figure 7 is an axial sectional view of figure 6,
- figure 8 is a perspective view of the base of the valve connector,
- figure 9 is an axial cross-sectional view and a view in larger scale of the base of the valve connector,
- figure 10 shows - in a larger scale - a detail of figure 9, and
- figure 11 a, b, c shows a radiography of a valve connector obtained by the Applicant according to the invention, in three successive conditions respectively deactivated (a), partially activated (b), fully activated (c).

### Detailed description of the invention

With reference to the drawings, the valve connector for infusion medical lines according to the invention consists of an axially elongated tubular body C which includes three components: a base 1, a lid 2 and an internal sealing elastic element 3. Typically, the base 1 and the lid 2 are made of moulded rigid plastic material, while the elastic element 3 is for example of silicone rubber.

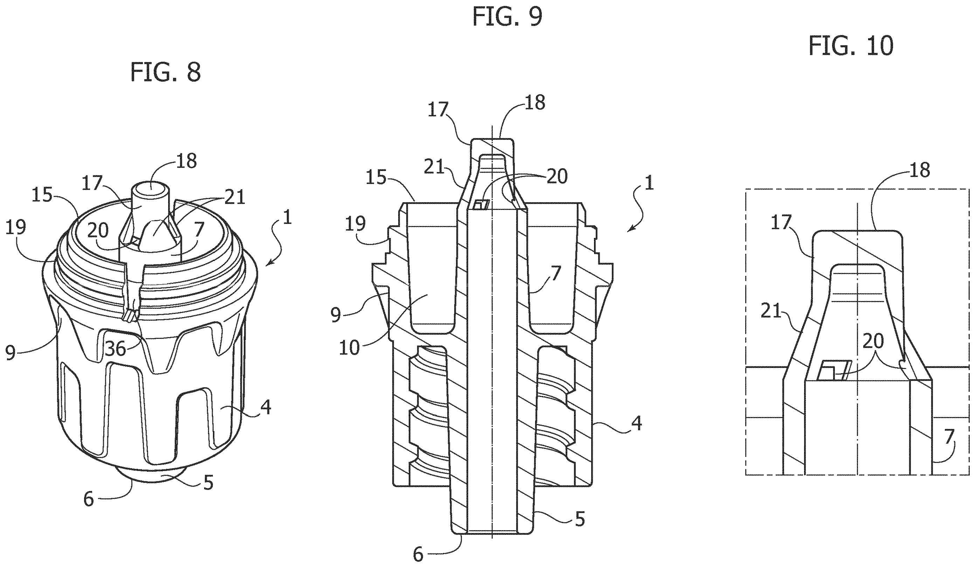

In the case of the shown example, the base 1 has a luer lock outer configuration with an internally threaded outer jacket 4 and an internal axial tang 5 with an outer conical surface which defines the outlet 6 of the connector. On the side opposite to the outlet 6 the axial tang 5 extends into the lid 2 with a hollow pin 7 better visible in figures 8-10. The axial length of the hollow pin 7 is limited, for the reasons clarified hereinafter, and it has - on the side opposite to the outlet 6 - a terminal with reduced diameter 17 closed by a radial wall 18. As better visible in figures 8 and 10, the terminal of the reduced diameter 17 has - at the base thereof - three radial slots 20 that are angularly equidistant from each other and separated by slide-like external intermediate protrusions 21.

The jacket 4 of the base 1 extends on the side opposite to the outlet 6 with a part 9 which surrounds most of the hollow pin 7, delimiting an annular space 10 therewith. The end edge of the part 9 has a step-like shape 19, traversed by a pair of diametrically opposite axial grooves 36, for anchoring and joining the lid 2 through a widened axial appendage 11 thereof.

The lid 2 has an inlet end 12 shaped to form a female Luer-Lock connection member for engagement, in a generally conventional fashion, with a male Luer-Lock connection member of a fluid introducer - not shown - for example consisting of a needleless syringe. The reduced axial length of the hollow pin 7 is such that the valve connector according to the invention may be advantageously used with fluid introducers which internally have a length beyond the maximum tolerance provided for, and also with luer slip-type syringes.

The inlet end 12 of the lid 2 is connected to a generally cylindrical intermediate tubular portion 13 joined to the widened axial appendage 11 through a radial flange 14. Such radial flange 14 is arranged axially facing a terminal axial protrusion 15 of the base 2 so as to delimit - therewith - a retaining annular seat 16 whose function will be clarified hereinafter.

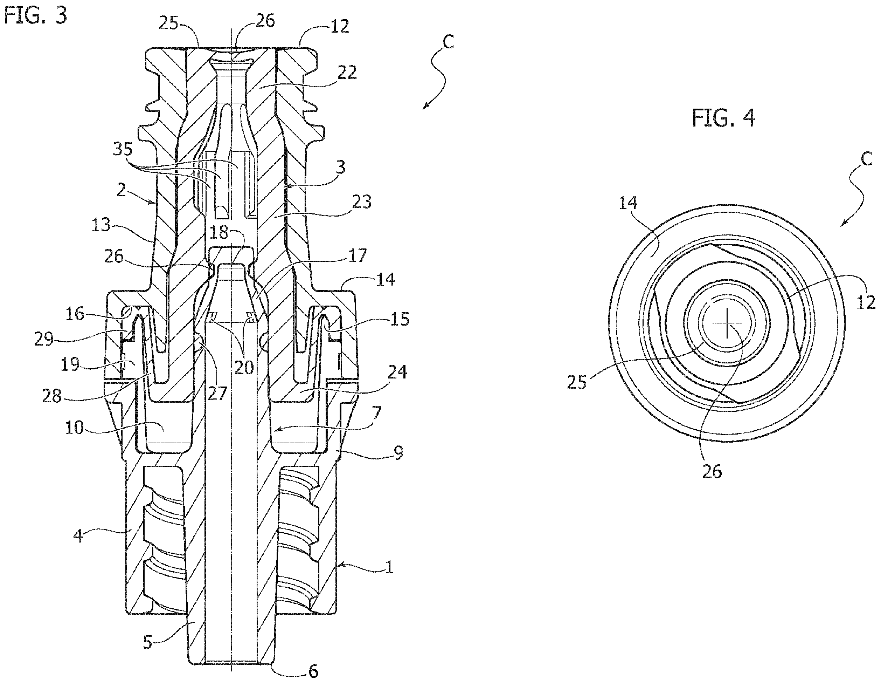

The elastic element 3 comprises, in a single piece, a hollow elastic head 22, a tubular stem 23 and a coupling base 24. Its general shape, and in particular its outer shape, substantially corresponds to the inner one of the lid 2, in which it is housed. Therefore, the elastic head 22 has a cylindrical outer surface complementary to that of the inner surface of the inlet end 12 so as to house it therein, as shown in figure 3, substantially without radial clearance that is with interference, in a closing condition in which such end 22 terminates with a terminal radial wall 25 flush with the inlet end 12.

Through the terminal wall 25 there is formed a pre-slit or radial engraving 26 which is kept closed in the closing condition of the elastic head 22 in the inlet end 12. In such condition there is obtained watertight anti-bacterial protection barrier between the internal of the valve connector and the external, while - at the same time - ensuring the possibility of an effective cleaning conventionally carried out using a cotton ball soaked with disinfectant.

The tubular stem 23 is internally formed with a circumferential series of axial grooves 35 and with a pair of reliefs or internal sealing rings 26, 27 axially spaced apart from each other and arranged in sliding sealing contact against the surface of the hollow pin 7. In detail, the sealing ring 26, having a smaller diameter, is located in the area of the tang 17 and, in the deactivated condition that is the condition for closing the connector shown in figure 3, upstream of the slots 20 with respect to the flow direction from the inlet 12 to the outlet 6. The other sealing ring 27, with smaller diameter, is located downstream of the slots 20.

The coupling base 24 of the elastic element 3 has a lapel or convolute 28 having a low thickness and terminating with a folded edge 29 inserted and locked in the annular seat 16 comprised between the radial portion 14 of the lid 2 and the terminal axial protrusion 15 of the base 1.

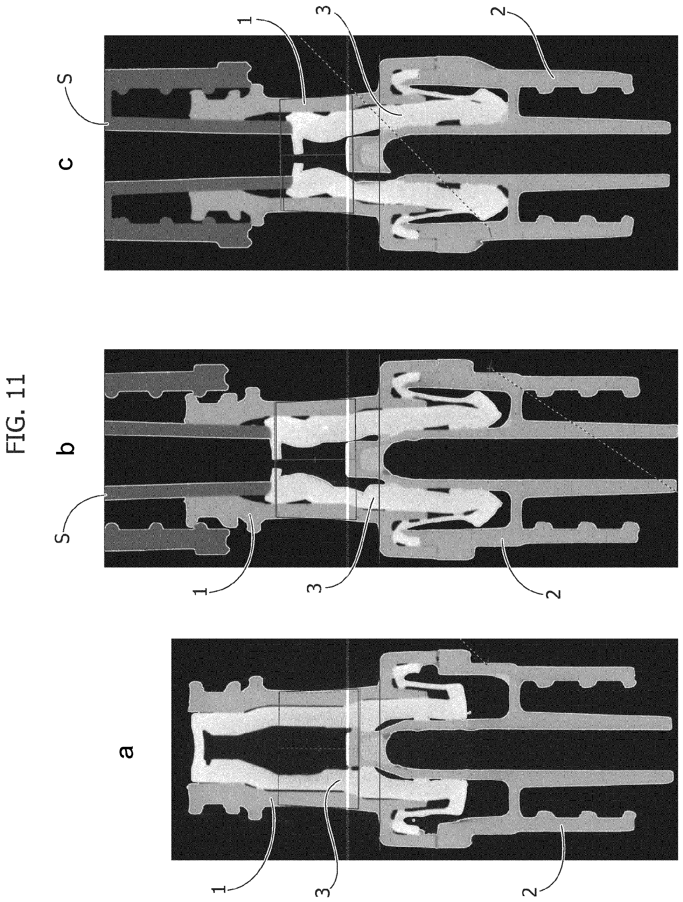

In use, when the tubular connector is not activated, the head 22 of the elastic element 3 hermetically seals the inlet end 12 and the ring 26 sealingly insulates the head 22 from the slots 20 and therefore from the outlet 6. Basically, in this condition, the elastic ring 26 and the elastic ring 27 act as a further double safety hermetic sealing of the connector. This condition is also realistically shown in the radiography representation of figure 11a which represents, similarly to figures 11b and 11c, a radiography of a valve connector made by the Applicant according to the invention.

When the end of a needleless syringe or of a Luer cone introducer (indicated with S in figures 11b and 11c) is frontally placed against the elastic head 22 and therefore inserted into the inlet end 12, as shown in the radiography of figure 11b, the convolute 28 of the coupling base 24 is deformed by traction starting to extend elastically and allowing the lower part of the tubular stem 23 to progressively penetrate into the annular space 10 of the base 1. The elastic head 22 is therefore displaced axially towards the internal of the connector, being disengaged from the inlet end 12 and allowing the dilatation and the ensuing opening of the pre-slit 26. The simultaneous translation of the tubular stem 23 of the elastic element 3 causes a corresponding displacement towards the outlet 6 of the sealing ring 26 which, sliding on the slides 21 of the tang 17 of the hollow pin 7, is arranged downstream of the slots 20 therefore opening the communication between the inlet end 12 and the outlet 6 as shown in figure 11b.

Continuing the insertion of the introducer S, the convolute 28 continues to stretch until substantially the entire annular space 10 is occupied on the lower part of the tubular stem 23, as shown in figure 11c. At the end of the insertion, the flow opening is maximum from the inlet end 12, through the fully open pre-slit 26 and the fully vacant slots 20, to the outlet 6.

The peculiar shape of the convolute 28 of the coupling base 24 of the elastic element 3 ensures, during the step for activating the valve connector, the most correct elastic deformation thereof without "buckling" effects.

It should be observed that in such condition the elastic head 22 remains axially spaced from the terminal wall 18 of the tang 17 of the hollow pin 7, which advantageously allows, as mentioned above, to use the valve connector according to the invention even with particularly long introducers.

When the introducer S is removed from the inlet end 12, the elastic return of the convolute 28 promptly restores the closing configuration of the valve connector in which the elastic head 22 returns into the inlet end 12 closing the pre-slit 26 again and the sealing ring 26 is once again arranged upstream of the slots 20 (Figure 11a).

Obviously, the construction details and the embodiments may widely vary with respect to what has been described and illustrated, without departing from the scope of protection of the present invention as defined in the claims.

## Claims

1. Valve connector for medical infusion lines, comprising:
- an axially elongated tubular body (C) consisting of a base (1) and a lid (2) coaxially joined together and defining an inlet (12) and an outlet (6), the base being coaxially formed with a hollow internal pin (7) communicating with the outlet (6) and having a closed terminal (17) projecting into the lid and having a lateral passage (20) adapted to be placed in communication with the inlet (12),
- an elastic element (3) inserted coaxially along said lid (2) and including:
- an elastic head (22) having a radial pre-slit (26) and normally arranged in a closing condition within said inlet (12) in which said pre-slit is closed, said elastic head (22) being axially displaceable towards said closed terminal (17) of the hollow pin (7), following the insertion of a fluid introducer (S) into said inlet (12), to allow said elastic head (22) to take a condition for opening said pre-slit (26), the elastic head (22) in the condition for opening said pre-slit (26) remaining axially spaced from said closed terminal (17) of the hollow pin (7) of the base (1),
- a tubular stem (23) joined to said head (22) and having an internal annular gasket (26) in sliding contact with said hollow pin (7) to control the communication between said lateral passage (20) of the closed terminal (17) of the hollow pin (7) and the inlet (12),
- an end retaining part (24) having an end edge (29) locked between said base (1) and said lid (2) and joined to said tubular stem (23) through a section (28) which is elastically deformable under traction during the axial displacement of said elastic head (22) from said closing condition towards said opening condition,
- the lateral passage of said closed terminal (17) consists of a plurality of radial slots (20) separated from each other by external slides (21),
**characterised in that**:
- said closed terminal (17) has a reduced diameter,
- said external slides (21) are slide-like external intermediate protrusions cooperating with said internal annular gasket (26) of the tubular stem (23) of the elastic element (3) to open the communication between the inlet (12) and the outlet (6) of the connector when said elastic head (22) moves to said opening condition,
- said section (28) of the elastic element (3) is formed, even in said closing condition, with a convolute which - during the displacement of said elastic head (22) from said closing condition towards said opening condition - deforms under traction within an annular space (10) of said base (1).

2. Valve connector according to claim 1, **characterised in that** said radial slots (20) are three.

3. Valve connector according to claim 1 or 2, **characterised in that** said tubular stem (23) of the elastic element (3) is internally formed with a circumferential series of axial grooves (35).

## Patentansprüche

1. Ventilverbinder für medizinische Infusionsleitungen, umfassend:
- einen axial länglichen röhrenförmigen Körper (C), der aus einer Basis (1) und einem Deckel (2) besteht, die koaxial miteinander verbunden sind und einen Einlass (12) und einen Auslass (6) definieren, wobei die Basis koaxial mit einem hohlen inneren Stift (7) ausgebildet ist, der mit dem Auslass (6) in Verbindung steht und einen geschlossenen Anschluss (17) aufweist, der in den Deckel vorsteht und einen seitlichen Durchgang (20) aufweist, der dazu ausgelegt ist, mit dem Einlass (12) in Verbindung gebracht zu werden,
- ein elastisches Element (3), das koaxial entlang des Deckels (2) eingesetzt ist und Folgendes umfasst:
- einen elastischen Kopf (22), der einen radialen Vorschlitz (26) aufweist und normalerweise in einem Schließzustand innerhalb des Einlasses (12) angeordnet ist, in dem der Vorschlitz geschlossen ist, wobei der elastische Kopf (22) nach dem Einsetzen einer Fluideinführvorrichtung (S) in den Einlass (12) in Richtung des geschlossenen Anschlusses (17) des hohlen Stifts (7) axial verschiebbar ist, um zu ermöglichen, dass der elastische Kopf (22) einen Zustand zum Öffnen des Vorschlitzes (26) einnimmt, wobei der elastische Kopf (22) in dem Zustand zum Öffnen des Vorschlitzes (26) von dem geschlossenen Anschluss (17) des hohlen Stifts (7) der Basis (1) axial beabstandet bleibt,
- einen röhrenförmigen Schaft (23), der mit dem Kopf (22) verbunden ist und eine innere ringförmige Dichtung (26) in Gleitkontakt mit dem hohlen Stift (7) aufweist, um die Verbindung zwischen dem seitlichen Durchgang (20) des geschlossenen Anschlusses (17) des hohlen Stifts (7) und dem Einlass (12) zu steuern,
- ein Endhalteteil (24), das eine Endkante (29) aufweist, die zwischen der Basis (1) und dem Deckel (2) verriegelt ist und mit dem röhrenförmigen Schaft (23) durch einen Abschnitt (28) verbunden ist, der unter Zug während der axialen Verschiebung des elastischen Kopfes (22) von dem Schließzustand in Richtung des Öffnungszustands elastisch verformbar ist,
- der seitliche Durchgang des geschlossenen Anschlusses (17) aus einer Vielzahl von radialen Schlitzen (20) besteht, die durch äußere Gleitstücke (21) voneinander getrennt sind,
**dadurch gekennzeichnet, dass**:
- der geschlossene Anschluss (17) einen verringerten Durchmesser aufweist,
- die äußeren Gleitstücke (21) gleitartige äußere Zwischenvorsprünge sind, die mit der inneren ringförmigen Dichtung (26) des röhrenförmigen Schafts (23) des elastischen Elements (3) zusammenwirken, um die Verbindung zwischen dem Einlass (12) und dem Auslass (6) des Verbinders zu öffnen, wenn sich der elastische Kopf (22) in den Öffnungszustand bewegt,
- der Abschnitt (28) des elastischen Elements (3) auch in dem Schließzustand mit einer Konvolution ausgebildet ist, die sich - während der Verschiebung des elastischen Kopfes (22) von dem Schließzustand in Richtung des Öffnungszustands - unter Zug innerhalb eines ringförmigen Raums (10) der Basis (1) verformt.

2. Ventilverbinder nach Anspruch 1, **dadurch gekennzeichnet, dass** die radialen Schlitze (20) drei sind.

3. Ventilverbinder nach Anspruch 1 oder 2, **dadurch gekennzeichnet, dass** der röhrenförmige Schaft (23) des elastischen Elements (3) innen mit einer Umfangsreihe von axialen Nuten (35) ausgebildet ist.

## Revendications

1. Connecteur de valve pour lignes médicales de perfusion, comprenant :
un corps tubulaire (C) axialement allongé se composant d'une base (1) et d'un couvercle (2) unis entre eux d'une manière coaxiale et définissant une entrée (12) et une sortie (6), la base étant formée, de manière coaxiale, avec une broche interne creuse (7) communiquant avec la sortie (6) et ayant une extrémité terminale fermée (17) faisant saillie dans le couvercle et ayant un passage latéral (20) adapté pour être placé en communication avec l'entrée (12),
un élément élastique (3) inséré de manière coaxiale le long dudit couvercle (2) et comprenant :
une tête élastique (22) ayant une pré-fente radiale (26) et normalement agencée dans une condition de fermeture dans ladite entrée (12) dans laquelle ladite pré-fente est fermée, ladite tête élastique (22) étant axialement déplaçable vers ladite extrémité terminale fermée (17) de la broche creuse (7), suite à l'insertion d'un dispositif d'introduction de fluide (S) dans ladite entrée (12), pour permettre à ladite tête élastique (22) d'adopter une condition pour ouvrir ladite pré-fente (26), la tête élastique (22) dans la condition d'ouverture de ladite pré-fente (26) restant axialement espacée de ladite extrémité terminale fermée (17) de la broche creuse (7) de la base (1),
une tige tubulaire (23) assemblée à ladite tête (22) et ayant un joint annulaire interne (26) en contact coulissant avec ladite broche creuse (7) pour contrôler la communication entre ledit passage latéral (20) de l'extrémité terminale fermée (17) de la broche creuse (7) et l'entrée (12),
une partie de retenue d'extrémité (24) ayant un bord d'extrémité (29) bloqué entre ladite base (1) et ledit couvercle (2) et assemblé à ladite tige tubulaire (23) par une section (28) qui est élastiquement déformable sous la traction pendant le déplacement axial de ladite tête élastique (22) de ladite condition de fermeture vers ladite condition d'ouverture,
le passage latéral de ladite extrémité terminale fermée (17) se compose d'une pluralité de fentes radiales (20) séparées les unes des autres par des glissières externes (21),
**caractérisé en ce que** :
ladite extrémité terminale fermée (17) a un diamètre réduit,
lesdites glissières externes (21) sont des saillies intermédiaires externes en forme de glissière coopérant avec ledit joint annulaire interne (26) de la tige tubulaire (23) de l'élément élastique (3) pour ouvrir la communication entre l'entrée (12) et la sortie (6) du connecteur, lorsque ladite tête élastique (22) se déplace dans ladite condition d'ouverture,
ladite section (28) de l'élément élastique (3) est formée, même dans ladite condition de fermeture, avec une convolution qui - pendant le déplacement de ladite tête élastique (22) de ladite condition de fermeture vers ladite condition d'ouverture - se déforme sous l'effet de la traction dans un espace annulaire (10) de ladite base (1).

2. Connecteur de valve selon la revendication 1, **caractérisé en ce que** lesdites fentes radiales (20) sont au nombre de trois.

3. Connecteur de valve selon la revendication 1 ou 2, **caractérisé en ce que** ladite tige tubulaire (23) de l'élément élastique (3) est formée, de manière interne, avec une série circonférentielle de rainures axiales (35).
